# FASCICULE DE BREVET EUROPEEN

(11) **EP 1 722 864 B1**
(45) Date de publication et mention de la délivrance du brevet: **23.07.2008**
(21) Numéro de dépôt: 05717652.1
(22) Date de dépôt: 04.01.2005
(51) Int. Cl.: A61Q 19/08, A61K 8/97

(54) **UTILISATION D'UN EXTRAIT D'ACMELLA OLERACEA POUR SON EFFET BOTOX-LIKE DANS UNE COMPOSITION COSMETIQUE ANTI-RIDES**
VERWENDUNG EINES ACMELLA OLERACEA EXTRAKTS WEGEN SEINER BOTOX-ARTIGEN WIRKUNG IN EINER KOSMETISCHEN ZUSAMMENSETZUNG GEGEN FALTENBILDUNG
USE OF AN ACMELLA OLERACEA EXTRACT FOR THE BOTOX-LIKE EFFECT THEREOF IN AN ANTI-WRINKLE COSMETIC COMPOSITION

(30) Priorité: 15.01.2004 FR 0450093
(43) Date de publication de la demande: 22.11.2006
(73) Titulaire: GATTEFOSSE SAS, 69804 Saint Priest Cedex (FR)
(72) Inventeur: DEMARNE, Frédéric Le Lusse 2 Résidence la Cadenell, F-13008 MARSEILLE (FR); PASSARO, Ghislaine, F-38340 VOREPPE (FR)
(74) Mandataire: Vuillermoz, Bruno
(86) Numéro de dépôt international: PCT/FR2005/050005
(87) Numéro de publication internationale: WO 2005/072698

(56) Documents cités:
- EP-A- 1 352 640
- US-B1- 6 387 398
- PATENT ABSTRACTS OF JAPAN vol. 010, no. 078 (C-335), 27 mars 1986 (1986-03-27) & JP 60 215610 A (TAKASAGO KORYO KOGYO KK), 29 octobre 1985 (1985-10-29)
- DATABASE BIOSIS [Online] BIOSCIENCES INFORMATION SERVICE, PHILADELPHIA, PA, US; avril 2002 (2002-04), CHAKRABORTY A ET AL.: "Local anaesthetic effect of Spilanthes axmella in experimental animal models" XP009035381 Database accession no. PREV200400023721
- "Food and drink additive - contg spilanthes acmella ext" DERWENT, 1973, XP002290410
- DATABASE BIOSIS [Online] BIOSCIENCES INFORMATION SERVICE, PHILADELPHIA, PA, US; 1984, HERDY G V H ET AL: "EFFECT OF SPILANTHOL JAMBU SPILANTHES-OLERACEA EXTRACT ON THE ACTION POTENTIAL BY RECORDING OF AN ATRIAL FIBER" XP002293685 Database accession no. PREV198580052356 & ARQUIVOS BRASILEIROS DE CARDIOLOGIA, vol. 43, no. 6, 1984, pages 423-428, ISSN: 0066-782X
- STASHENKO E E, ET AL.: "Volatile secondary metabolites from Spilanthes americana obtained by simultaneous steam distillation-solvent extraction and supercritical fluid extraction" JOURNAL OF CHROMATOGRAPHY, vol. 752, 1996, pages 223-232, XP002293687
- ANSARI A H, ET AL.: "Analgesic study of N-Isobutyl-4,5-Decadienamide Isolated from the Flowers of Spilanthes Acmella (Murr)" INDIAN JOURNAL OF PHARMACEUTICAL SCIENCES, vol. 50, no. 2, 1988, page 106, XP009035382
- GREGER H: "Alkamides: Structural Relationships, Distribution and Biological Activity" PLANTA MEDICA, 1984, pages 366-375, XP009035401
- GREGER H ET AL.: "New Amides from Spilanthes oleracea" MONATSHEFTE FÜR CHEMIE, vol. 116, 1985, pages 273-277, XP009035385
- MARTIN R., AND BECKER H.: "Spilanthol-related amides from Acmella ciliata" PHYTOCHEMISTRY, vol. 23, no. 8, 1984, pages 1781-1783, XP002293684 GREAT BRITAIN

## Description

L'invention concerne l'utilisation de spilanthol comme antirides, en particulier sous la forme d'un extrait d'*Acmella o*/*eracea*, par son activité botox like, c'est-à-dire par sa capacité à inhiber la contraction des muscles sous-cutanés du visage.

Le botox^{®} (marque déposée par la société ALLERGAN Inc) ou toxine botulique est une substance extraite de la bactérie dénommée *Clostridium botulinum*. Cette toxine injectée dans les muscles du visage paralyse ceux-ci, ce qui conduit à atténuer les rides faciales. On a notamment constaté que le botox^{®} était particulièrement efficace pour atténuer les rides de froncement de sourcils, celles du front, les pattes d'oie et les rides de plissement du nez. Néanmoins, l'inconvénient essentiel du botox^{®} reste sa toxicité puisque son utilisation dans certaines conditions entraîne la mort.

En d'autres termes, le problème posé est de développer un produit qui présente les mêmes propriétés que le botox^{®} (botox like), mais dénué de toute toxicité.

Dans le cadre de sa recherche, le Demandeur a constaté que le spilanthol, en particulier lorsqu'il se présente sous la forme d'un extrait d'*Acmella oleracea,* était capable d'inhiber efficacement l'activité contractile des muscles sous-cutanés du visage.

*Acmella oleracea* est une plante du genre Acmella appartenant à la famille des *Compositoe-He*/*iantheoe* identifiés sous le numéro EINECS 290335-0 et CAS 90131-24-1.

Cette plante est également connue sous les dénominations suivantes : *Spilanthus oleracea, Pyrethrum spilanthus,* mais également *Spilanthes acmella* variété *oleracea.*

*Acmella oleracea* est une petite plante annuelle, originaire d'Amérique du Sud de 40 à 60 cm de hauteur. Cette plante fragile fleurit toute l'année en donnant de nombreuses fleurs jaunes. La plante se multiplie facilement par graines et par boutures.

*Acmella oleracea* est notamment exploitée au sein de compositions cosmétiques, comme source d'une molécule désignée spilanthol.

Le document JP 9175947 décrit ainsi une composition pour la repousse des cheveux comprenant un extrait de *Spilanthes oleracea* en tant que produit actif mélangé dans une matière grasse, en particulier une huile.

Le document JP 6072858 décrit une composition à base de spilanthol utilisée comme bain moussant pour son effet rafraîchissant de la peau.

Le document JP60215610 décrit une préparation pour le bain présentant des propriétés sédatives mais également raffermissantes.

Le document US-B-6 387 398 décrit l'utilisation cosmétique d'un extrait de *Spilanthes acmella,* notamment comme agent désodorisant, pour la sensation de fraîcheur apportée par le *Spilanthol.*

Le document EP-A-3 532 640 concerne l'utilisation de *Acmella oleracea* (*Gin-New-Kou*) uniquement pour l'effet inhibiteur de la formation de mélanine avec comme application, l'indication anti-âge.

La publication CHAKRABORTY « local anaesthetic effect of *Spilanthes acmella* in experimental animal models » (XP009035381) divulgue l'effet anesthésiant local de *Spilanthes acmella.*

La publication HERDY « effect of spilanthol jambu spilanthes oleracea extract on the action potential by recording of an atrial fiber » (XP002293685) met en exergue la capacité du *Spilanthol* à générer une arythmie lorsqu'il est injecté dans des coeurs de lapins isolés.

Le document STASHENKO « volatile secondary metabolites from *Spilanthes americana* obtained by simultaneous steam distillation-solvant extraction and supercritical fluid extraction » (XP002293687) décrit un procédé d'extraction des métabolites de *Spilanthes américana*. Dans l'introduction, il est indiqué que cette plante possède des effets analgésique et paralysant, notamment lorsqu'elle est appliquée sur la langue.

A la connaissance du Demandeur, aucun document de l'état de la technique ne décrit la capacité du spilanthol et au surplus, celle d'un extrait *Acmella oleracea* à inhiber la contraction des muscles sous-cutanés, en particulier ceux du visage.

Bien entendu, pour cette application, le spilanthol peut être utilisé localement par voie topique ou par injection directement au niveau des muscles sous-cutanés.

Comme déjà dit, le Demandeur a constaté que l'utilisation comme source de spilanthol, d'un extrait d'*Acmella oleracea* donnait encore de meilleurs résultats sur la contraction des muscles sous-cutanés que le spilanthol pur. En tant qu'extrait, on utilise en pratique les parties aériennes, avantageusement les feuilles ou les boutons floraux.

Compte-tenu de cette propriété sur les fibres musculaires mise en évidence dans la présente demande, l'invention concerne également l'utilisation de spilanthol pur ou sous forme d'un extrait végétal, en particulier d'*Acmella oleracea* pour la fabrication d'une composition cosmétique anti-rides.

Lorsque le spilanthol se présente sous forme pure, la concentration de spilanthol dans la composition est comprise entre 0,005 et 10 % en poids, avantageusement entre 0,05 et 5 % en poids de la composition.

L'extrait végétal utilisé comme source de spilanthol peut, quant à lui, se présenter sous forme sèche ou liquide. Lorsqu'il se présente sous forme sèche, il représente entre 0,005 et 20 %, avantageusement entre 0,1 et 10 % en poids de la composition. Lorsqu'il se présente sous forme liquide, il représente entre 0,1 et 20 %, avantageusement entre 0,5 et 10 % en poids de la composition.

L'extraction est effectuée à partir de la plante entière ou d'une partie de plante, en particulier les boutons floraux. La plante ou partie de plante est broyée dans un solvant polaire utilisable dans les applications cosmétiques topiques, donc en milieux aqueux, alcoolique ou glycolique. Généralement, le solvant polaire est choisi dans le groupe comprenant l'eau, l'éthanol, les glycols tels que propylène-glycol, butylène-glycol, seuls ou en mélange, l'éthanol restant cependant l'un des solvants préférés.

La composition selon l'invention peut se présenter sous toutes les formes galéniques normalement utilisées pour une application topique sur la peau, notamment sous forme d'une solution aqueuse, d'une émulsion huile-dans-eau ou eau-dans-huile ou multiple, d'une émulsion siliconée, d'une microémulsion ou nanoémulsion, d'un gel aqueux.

Cette composition peut être plus ou moins fluide et avoir l'aspect entre autre d'une crème blanche ou colorée, d'une pommade, d'un lait, d'une lotion, d'un sérum, d'un gel.

La composition de l'invention peut contenir les adjuvants habituels dans les domaines cosmétique et dermatologique, tels que les matières grasses, les émulsionnants et coémulsionnants, les gélifiants hydrophiles ou lipophiles, les actifs hydrophiles ou lipophiles, les conservateurs, les antioxydants, les solvants, les parfums, les charges, les filtres hydrophiles et lipophiles, les matières colorantes, les neutralisants, les agents propénétrants, et les polymères.

Les quantités de ces différents adjuvants sont celles classiquement utilisées dans les domaines considérés, et par exemple de 0.01 à 30% du poids total de la composition. Ces adjuvants, selon leur nature, peuvent être introduits dans la phase grasse ou dans la phase aqueuse.

Comme matières grasses utilisables dans l'invention, on peut utiliser les huiles minérale, les huiles d'origine animale (lanoline), les huiles de synthèse (isopropyl myristate, octyldodecyl, isostearyl isostearate, decyl oleate, isopropyl palmitate), les huiles siliconées (eyclomethicone, dimethicone) et les huiles fluorées. On peut utiliser comme matières grasses des alcools gras, des acides gras, des cires et des gommes et en particulier les gommes et élastomères de silicone.

Comme émulsionnants et coémulsionnants utilisables dans l'invention, on peut citer par exemple les esters de polyglycérols et d'acide gras, les esters de sucrose et d'acide gras, les esters de sorbitane et d'acide gras, les esters d'acide gras et de sorbitane oxyéthylénés, les ethers d'alcool gras et de PEG, les esters de glycérol et d'acide gras, les alkyl sulfates, les alkyl éther sulfates, les alkyl phosphates, les alkyl polyglucosides, les diméthicone copolyols.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, les argiles et les copolymères d'acide 2-acrylamido-2-méthylpropane.

Comme gélifiants lipophiles, on peut citer les argiles modifiées comme les bentones, les sels métalliques d'acides gras, la silice hydrophobe et l'éthylcellulose.

La composition cosmétique peut également contenir des actifs. Comme actifs, on peut utiliser notamment les dépigmentants, les émollients, les hydratants, les anti-séborrhéiques, les anti-acnéiques, les agents kératolytiques et/ou desquamants, les agents drainants, les agents anti-irritants, les agents apaisants, les amincissants tels que les bases xanthiques (caféine), les vitamines et leurs mélanges, les agents matifiants, les actifs anti-âge tel que le rétinol et les agents anti-rides.

En cas d'incompatibilité entre eux ou avec l'extrait d'*Acmella oleracea,* les actifs indiqués ci-dessus et/ou l'extrait d'*Acmella oleracea* peuvent être incorporés dans des sphérules, notamment des vésicules ioniques ou non-ioniques et/ou des nanoparticules (nanocapsules et/ou nanosphères), de manière à les isoler les uns des autres dans la composition.

Comme conservateurs utilisables selon l'invention, on peut citer l'acide benzoïque, ses sels et ses esters ; l'acide sorbique et ses sels ; les parabens, leurs sels et esters ; le triclosan ; l'imidazolidinyl urée ; le phénoxyéthanol ; la DMDM hydantoïne ; le diazolidinyl urée ; la chlorphenesin.

Comme antioxydants utilisables selon l'invention, on peut citer les agents chelatants tels que l'EDTA et ses sels.

Comme solvants utilisables selon l'invention, on peut citer, comme déjà dit, l'eau, l'éthanol, la glycérine, le propylène glycol, le butylène glycol, le sorbitol.

Comme charges utilisables selon l'invention, on peut citer le talc, le kaolin, le mica, la serecite, le magnésium carbonate, l'aluminium silicate, le magnésium silicate, les poudres organiques telles que le nylon.

Comme filtres utilisables selon l'invention, on peut citer les filtres UVA et UVB classiquement utilisés tels que la benzophénone-3, le butyl méthoxydibenzoyl méthane, l'octocrylène, l'octyl méthoxycinnamate, le 4-méthylbenzylidene camphor, l'octyl salycylate, le tacephthalylidene dicamphor sulfanic acid, et le drométrizole trisiloxane. On citera également les filtres physiques TiO₂ et ZnO sous leurs formes micrométriques et nanométriques.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Comme neutralisants utilisables selon l'invention, on peut citer la soude, la triethanolamine, l'aminométhyl propanol, l'hydroxyde de potassium.

Comme agents propénétrants utilisables selon l'invention, on peut citer les alcools et glycols (éthanol, propylène glycol), l'éthoxydiglycol, les alcools et acides gras (acide oléique), les esters d'acides gras, le diméthyl isosorbide.

L'invention concerne également l'utilisation de la composition contenant le spilanthol pur ou sous forme d'extrait *d'Acmella oleracea,* comme anti-rides. Elle se rapporte également au traitement cosmétique des rides par application locale ou par voie sous cutanée, d'une quantité efficace d'une composition cosmétique à base de spilanthol pur ou sous forme d'extrait d'*Acmella oleracea.*

L'invention et les avantages qui en découlent ressortiront bien des exemples de réalisation suivants à l'appui des figures annexées.

### Exemple 1 : Fabrication d'un extrait d'Acmella oleracea

La plante entière séchée provient d'Amérique du Sud ou plus généralement de pays tropicaux. Cette plante est broyée jusqu'à obtention d'une poudre.

L'extraction de la plante broyée est réalisée dans un mélange éthanol 96,2° H₂O (80/20) ; volume/volume à température ambiante sous agitation magnétique à l'abri de la lumière, durant 6 heures.

L'extrait est ensuite filtré sur filtre nylon puis sur membrane de cellulose (jusqu'à 0,22 microns). Dans l'exemple 2, il est ensuite lyophilisé pour être utilisé dilué à 50 % dans de la malto-dextrine.

### Exemple 2 :

### 1. Objectif de l'étude

Evaluer la capacité de spilanthol purifié et d'un extrait *d'Acmella oleracea* à induire un blocage réversible des contractions musculaires.

### 2. Méthodologie générale

### Modèle nerf-muscle

La co-culture nerf-muscle est un modèle de culture qui permet de recréer une innervation des cellules musculaires striées humaines avec des explants de moelle épinière et de ganglions rachidiens d'embryons de rat. Après 21 jours de culture, les fibres musculaires formées se contractent spontanément.

Le modèle de coculture nerf-muscle est un modèle adapté pour étudier l'influence d'un produit sur la fréquence des contractions musculaires mais aussi pour étudier la récupération de l'activité contractile après blocage des contractions musculaires par un produit.

Le carisoprodol à 1 mM est utilisé en tant que contrôle positif de blocage réversible des contractions musculaires.

### Mesure des fréquences de contraction

Pour chaque puits de culture sélectionné, une fibre musculaire montrant des contractions régulières est référencée.
Grâce à un logiciel de comptage automatisé le nombre de contractions est comptabilisé pendant 30 secondes à chaque temps de mesure : avant incubation (fréquence pré-incubation), pendant l'incubation et durant la phase de récupération de l'activité contractile après élimination du produit.

### Interprétation des résultats

Chaque mesure étant réalisée en triplicate (dans 3 puits distincts), une activité est considérée comme significative lorsque au moins 2 fibres sur 3 présentent le même effet selon la gamme de modulation suivante :
- Lorsque la fréquence de contractions est supérieure à 120% par rapport à la fréquence de préincubation avant ajout du produit, nous parlons d'augmentation de fréquence de contractions, notée +. Lorsque la fréquence devient trop élevée pour être mesurable, on parle de vibration notée Vib.
- Lorsque la fréquence de contractions est comprise entre 80% et 120% par rapport à la fréquence de préincubation avant ajout du produit, elle est non modifiée et notée 0.
- Lorsque la fréquence de contractions est inférieure à 80% par rapport à la fréquence de préincubation avant ajout du produit, nous parlons de diminution de fréquence de contractions, notée - ou bloc (blocage) si elle est égale à 0%.

Nous parlons de récupération complète de l'activité contractile après blocage lorsque au moins 2 fibres sur 3 retrouvent une fréquence de contractions supérieure ou égale à 80 % de la fréquence de contractions de pré-incubation, noté +.

Nous parlons de récupération incomplète de l'activité contractile après blocage lorsque au moins 2 fibres sur 3 retrouvent une fréquence de contractions comprise entre 10 et 80% de la fréquence de contractions de pré-incubation, noté +/-.

### 3. Produits à l'étude

Nature et origine du produit :
- Extrait lyophilisé des parties aériennes (contenant les boutons floraux) d'*Acmella Oleracea* dilué à 50% dans de la malto-dextrine,
- Spilanthol purifié à 97% à partir d'un extrait d'*Acmella Oleracea.*

### 4. Déroulement de l'étude

### a/ Spilanthol pur

La fréquence de contraction est déterminée après 5 minutes, 1 heure et 6 heures d'incubation avec le produit. Au temps 6 heures, le produit est éliminé et la récupération de l'activité contractile est étudiée 1 heure et 24 heures après.

### b/ Extrait d'Acmella oleracea

La fréquence de contraction est déterminée (aux temps 5 minutes, 1 heure et 6 heures) jusqu'à obtention d'un blocage des contractions par le produit. Dès blocage, le produit est éliminé et la récupération de l'activité contractile est étudiée à 1 heure, 4 heures et 24 heures.

### 5. Résultats

### Spilanthol pur

| **Produit** | **[C}** | **Fréquence de contractions (%)** | | | | | **Interprétation** | | | | |
|---|---|---|---|---|---|---|---|---|---|---|---|
| | | Incubation sans lavage | | | Récupération après lavage | | Incubation sans lavage | | | Récupération après lavage | |
| | | 5 min | 1 h | 6 h | 1 h | 24 h | 5 min | 1 h | 6 h | 1 h | 24 h |
| Spilanthol pur | 40.10⁻⁵ % | 0 | 0 | 0 | 0 | 0 | bloc | bloc | bloc | bloc | bloc |
| | | 0 | 0 | 0 | 0 | 37 | bloc | bloc | bloc | bloc | +/- |
| | | 0 | 0 | 0 | 0 | 0 | bloc | bloc | bloc | bloc | bloc |
| | 160.10⁻⁵ % | 0 | 0 | 0 | 0 | 0 | bloc | bloc | bloc | bloc | bloc |
| | | 7 | 0 | 4 | 0 | 0 | - | bloc | - | bloc | bloc |
| | | 0 | 0 | 0 | 0 | 0 | bloc | bloc | bloc | bloc | bloc |

Aux concentrations (40.10⁻⁵ % et 160.10⁻⁵ %) le spilanthol pur bloque les contractions musculaires après 5 minutes d'incubation. Le blocage se maintient jusqu'au temps 6 heures et les fibres restent bloquées pendant 24 heures, après élimination du produit.

### Extrait d'Acmella oleracea

| **Produit** | **[C]** | **Fréquence de contractions (%)** | | | | | **Interprétation** | | | | |
|---|---|---|---|---|---|---|---|---|---|---|---|
| | | Incubation sans lavage | | | Récupération après lavage | | Incubation sans lavage | | | Récupération après lavage | |
| | | 5 min | 1 h | 6 h | 1 h | 4 h 24 h | 5 min | 1 h | 6 h | 1 h | 4 h 24 h |
| Extrait | 600.10⁻⁵ % | Vib | 0 | 0 | 114 | Non testé | + | bloc | bloc | + | Non testé |
| | | Vib | Vib | 0 | 171 | | + | + | bloc | + | |
| | | 400 | Vib | Vib | 194 | | + | + | + | + | |
| | 1200.10⁻⁵ % | 0 | Non testé | | 47 | Non testé | bloc | Non testé | | +/- | Non testé |
| | | 5 | | | 104 | | - | | | + | |
| | | 0 | | | 110 | | bloc | | | + | |
| | 2400.10⁻⁵ % | 63 | Non testé | | 97 | Non testé | - | Non testé | | + | Non testé |
| | | 0 | | | 145 | | bloc | | | + | |
| | | 0 | | | 126 | | bloc | | | + | |

A une concentration de 600.10⁻⁵%, l'extrait bloque les fréquences de contractions des fibres musculaires après 6 heures d'incubation. Après lavage des cultures, les fibres musculaires récupèrent totalement leur activité contractile en 1 heure.

Aux concentrations de 1200.10⁻⁵% et 2400.10⁻⁵%, l'extrait bloque les fréquences de contractions des fibres musculaires après 5 minutes d'incubation. Après lavage des cultures, les fibres musculaires récupèrent totalement leur activité contractile en 1 heure.

### 6. Conclusion

Dans les conditions de l'étude, l'extrait d'*Acmella Oleracea* et le composé spilanthol entraînent un blocage des contractions musculaires. Une récupération de l'activité contractile est observée en présence de l'extrait végétal mais non observée en présence de spilanthol. Cette différence doit probablement être attribuée à une différence de protocole puisque le système nerf-muscle a été incubé seulement 5 minutes en présence d'extrait contre 6 heures en présence de spilanthol pur.

L'effet anti-ride de la toxine botulique reposant sur sa capacité à inhiber la contraction des muscles sous-cutanés tenue pour responsable des rides d'expression (rides profondes), les produits testés possèdent, par leur capacité à inhiber l'activité contractile (ou effet botox-like), un potentiel anti-ride au même titre que la toxine botulique.

### Exemple 3 :

### Crème de jour anti-rides

| Composition | Quantité (%) |
|---|---|
| Steareth-21 | 2.00 |
| Steareth-2 | 3.00 |
| Acide stéarique | 1.00 |
| Cyclopentasiloxane | 3.00 |
| Myristate d'Octyldodécyle | 2.00 |
| Alcool cétylique | 1.00 |
| Stéarate de glycérol | 0.50 |
| Octyl Methoxycinnamate | 5.00 |
| Benzophenone-3 | 2.00 |
| Aluminium amidon octenylsuccinate | 3.00 |
| Phenoxyethanol, Methylparaben, Butylparaben, Ethylparaben, Propylparaben | 1.00 |
| Carbomer | 0.15 |
| Gomme xanthane | 0.30 |
| EDTA disodique | 0.05 |
| Glycérine | 3.00 |
| Hydroxyde de sodium (solution à 10%) | 0.30 |
| Extrait sec d'*Acmella oleracea* | 2.00 |
| Ethanol | 3.00 |
| Acétate de tocophérol | 0.50 |
| Parfum | 0.40 |
| Eau | Qsp 100 |

### Fond de teint

| Composition | Quantité (%) |
|---|---|
| Stéarate de Glycérol, Stéarate de Propylène Glycol, Isostéarate de Glycérol, Isostéarate de Propylène Glycol, Oleth-25, Ceteth-25 | 5 |
| Dibehenate de Glycérol, Tribehenin, Behenate de Glycérol | 1 |
| Ethoxydiglycol Oleate | 7.5 |
| Isostearate d'Isostearyl | 5 |
| Alcool cétéarilique | 2 |
| Dimethicone | 5 |
| Tocopheryl Acetate | 0.5 |
| Phenoxyethanol, Methylparaben, Ethylparaben, Propylparaben, Isobutylparaben | 0.6 |
| Gomme de xanthane | 0.4 |
| Cellulose Microcrystalline, Gomme de Cellulose | 1.5 |
| Dioxide de Titane | 6.6 |
| Oxydes métalliques (pigment jaune) | 1.55 |
| Oxydes métalliques (pigment rouge) | 0.43 |
| Oxydes métalliques (pigment noir) | 0.11 |
| Ethoxydiglycol Oleate | 2.5 |
| Dimethicone, Dimethiconol | 3 |
| Alcool | 5 |
| Extrait sec d'*Acmella oleracea* | 2 |
| Eau | Qsp 100 |

### Emulsion H/E

| Composition | Quantité (%) |
|---|---|
| Phenoxyethanol, Methylparaben, Butylparaben, Ethylparaben, Propylparaben | 1 |
| Carbomer | 0.4 |
| Glycérine | 3 |
| Gomme de xanthane | 0.1 |
| Polysorbate-60 | 0.9 |
| Glyceryl Stearate, PEG-100 Stearate | 2.1 |
| Cetyl Alcohol | 2.6 |
| Huile de paraffine | 7.5 |
| Isopropyl Myristate | 7.5 |
| Ethoxydiglycol | 5 |
| Extrait sec d'Acmella oleracea | 1 |
| Parfum | 0.2 |
| Triethanolamine | 0.3 |
| Eau | Qsp 100.0 |

### Emulsion E/H

| Composition | Quantité (%) |
|---|---|
| Glycerine | 3 |
| Propylene Glycol, Diazolidinyl Urée, Methylparaben, Propylparaben | 1 |
| Sulfate de Magnésium | 0.7 |
| Cetyl Dimethicone Copolyol | 2.5 |
| Isohexadecane | 5 |
| Triglycéride Caprylic/Capric | 5 |
| Dimethicone | 5 |
| Alcool | 5 |
| Extrait sec d'Acmella oleracea | 2 |
| Parfum | 0.1 |
| Eau | Qsp 100 |

### Microemulsion

| Composition | Quantité (%) |
|---|---|
| Glycérides PEG-8 Caprylic/Capric | 13.33 |
| Polyglyceryl-6 Dioleate | 8.67 |
| Isostearate d'Isostéaryl | 4 |
| Cyclomethicone | 2.3 |
| Diisopropyl Adipate | 1.6 |
| Octyldodecanol | 2 |
| PPG-5 Ceteth-20 | 2 |
| Phenoxyethanol, Methylparaben, Butylparaben, Ethylparaben, Propylparaben | 0.4 |
| Ethoxydiglycol | 2 |
| Extrait sec d'Acmella oleracea | 1 |
| Eau | Qsp 100 |

### Emulsion multiple W/O/W

| Composition | Quantité (%) |
|---|---|
| PEG-30 Dipolyhydroxystearate | 2.4 |
| Isohexadecane | 9 |
| PPG-15 Stearyl Ether | 4.5 |
| Triglycéride Caprylic/Caprue | 4.5 |
| Sulfate de Magnésium | 0.82 |
| Propylene Glycol, Diazolidinyl Urea, Methylparaben, Propylparaben | 1.2 |
| Extrait sec d'Acmella oleracea | 2 |
| Poloxamer 407 | 2 |
| Glycérine | 3 |
| Gomme de xanthane | 0.7 |
| Parfum | 0.2 |
| Eau | Qsp 100 |

## Revendications

1. Utilisation non-thérapeudique d'une composition contenant du spilanthol pur ou sous forme d'extrait d'*Acmella oleracea*, comme anti-rides par inhibition de la contraction des muscles sous-cutanés du visage.

2. Utilisation selon la revendication 1, **caractérisée en ce que** la source de spilanthol est un extrait d'*Acmella oleracea*.

3. Utilisation selon la revendication 2, **caractérisée en ce que** l'extrait est un extrait de boutons floraux.

4. Utilisation selon la revendication 1, **caractérisée en ce que** la concentration de spilanthol est comprise entre 0,005 et 10 % en poids, avantageusement 0,05 et 5 % en poids de la composition.

5. Utilisation selon la revendication 2, **caractérisée en ce que** lorsqu'il se présente sous forme sèche, l'extrait représente entre 0,005 et 20 % en poids de la composition.

6. Utilisation selon la revendication 2, **caractérisée en ce que** lorsqu'il se présente sous forme liquide, l'extrait représente entre 0,1 et 20 % en poids de la composition.

7. Utilisation d'une composition à base de spilanthol pur ou sous forme d'extrait d'*Acmella oleracea* pour la fabrication d'une composition thérapeutique anti-ride appliquée par voie sous cutanée en une quantité efficace.

## Claims

1. Non therapeutically use of a composition containing pure spilanthol or in plant extract form of *Acmella oleracea* as anti-wrinkle by inhibiting contractile activity in subcutaneous muscles.

2. Use as claimed in claim 1, **characterised in that** the source of spilanthol is an *Acmella oleracea* extract.

3. Use as claimed in claim 2, **characterised in that** the extract is an extract of flower buds.

4. Use as claimed in claim 1, **characterised in that** the concentration of spilanthol is between 0.005 and 10% of the weight, and advantageously 0.05 and 5% of the weight of the composition.

5. Use as claimed in claim 2, **characterised in that** when in dry form, the extract comprises between 0.005 and 20% of the weight of the composition.

6. Use as claimed in claim 2, **characterised in that** when in liquid form, the extract comprises between 0.1 and 20% of the weight of the composition.

7. Use of a composition containing pure spilanthol or in plant extract form of *Acmella oleracea* for the production of a therapeutic anti-wrinkles composition by subcutaneously applying an effective quantity of said composition.

## Patentansprüche

1. Nicht-therapeutische Verwendung einer Zusammensetzung, die Spilanthol pur oder in Form eines Acmella Oleracea Extraktes enthält, gegen die Faltenbildung durch Hemmung der Kontraktion der subkutanen Gesichtsmuskeln.

2. Verwendung gemäß Anspruch 1, **dadurch gekennzeichnet, dass** die Spilanthol-Quelle ein Acmella Oleracea Extrakt ist.

3. Verwendung gemäß Anspruch 2, **dadurch gekennzeichnet, dass** der Extrakt ein Extrakt aus Blütenknospen ist.

4. Verwendung gemäß Anspruch 1, **dadurch gekennzeichnet, dass** die Konzentration an Spilanthol zwischen 0,005 und 10 Gewichts-% umfasst, vorteilhafterweise 0,05 und 5 Gewichts-% der Zusammensetzung.

5. Verwendung gemäß Anspruch 2, **dadurch gekennzeichnet, dass** der Extrakt, wenn er in trockener Form vorliegt, zwischen 0,005 und 20 Gewichts-% der Zusammenstellung aufweist.

6. Verwendung gemäß Anspruch 2, **dadurch gekennzeichnet, dass**, der Extrakt, wenn er in flüssiger Form vorliegt, zwischen 0,1 und 20 Gewichts-% der Zusammensetzung aufweist.

7. Verwendung einer Zusammensetzung auf der Basis von Spilanthol pur oder in Form eines Acmella Oleracea Extrakts zur Herstellung einer therapeutischen Zusammensetzung gegen Faltenbildung, die auf subkutanem Weg in wirksamer Quantität angewendet wird.
